# EUROPÄISCHE PATENTANMELDUNG

(11) **EP 1 612 317 A2**
(43) Veröffentlichungstag der Anmeldung: **04.01.2006**
(21) Anmeldenummer: 05009561.1
(22) Anmeldetag: 30.04.2005
(51) Int. Cl.: D06F 35/00, D06F 37/42, A61L 2/00

(54) **Bakteriozid dotierte bzw. beschichtete Konstruktionsteile in Waschmaschinen, Wäschetrocknern oder dergleichen**

(30) Priorität: 30.06.2004 DE 102004031633
(71) Anmelder: Schott AG, 55122 Mainz (DE)
(72) Erfinder: Schultheis, Bernd, 55270 Schwabenheim (DE); Schaupert, Kurt, Dr., 65719 Hofheim (DE)
(74) Vertreter: Fleck, Hermann-Joseph

(57) **Zusammenfassung**

Die Erfindung betrifft ein Haushaltsgerät mit einem aus einzelnen Konstruktionsteilen zusammengesetzten, luft- und/oder wasserführendem System innerhalb dem eine Waschlauge und/oder ein Gasstrom führbar ist.

Die Desinfektion von wasser- bzw. luftführenden Teilen gelingt dann auf einfache Weise wenn vorgesehen ist, dass wenigstens eines der Konstruktionsteile zumindest teilweise an seiner dem laugeführenden Bereich zugewandten Oberfläche antimikrobiell oder bakteriozid beschichtet ist und/oder dass diese Oberfläche antimikrobiell oder bakteriozid dotiert ist bzw. wenigstens eines der Konstruktionsteile zumindest teilweise an seiner den luftführenden Bereich zugewandten Oberfläche antimikrobiell oder bakteriozid beschichtet ist und/oder dass diese Oberfläche antimikrobiell oder bakteriozid dotiert ist.

## Beschreibung

Die Erfindung betrifft ein Haushaltsgerät, insbesondere Wäschetrockner oder Waschmaschine mit einem aus einzelnen Konstruktionsteilen zusammengesetzten, luft- und/oder wasserführendem System innerhalb dem eine Waschlauge, ein Gasstrom oder dergleichen führbar ist.

Die Erfindung betrifft weiterhin einen Wäschetrockner mit einem aus einzelnen Konstruktionsteilen zusammengesetzten luftführendem System innerhalb dem Trockenluft führbar ist.

Waschmaschinen und/oder Wäschetrockner zeichnen sich heute insbesondere dadurch aus, dass diese besonders energiesparende Wasch- und Trockenprogramme aufweisen. Dies wird insbesondere durch ein Absenken der Prozesstemperatur erreicht. So sind beispielsweise Waschtemperaturen mit 30°C bis 40°C üblich, die zwar in Verbindung mit den verfügbaren Waschmitteln recht gute Waschergebnisse zeigen, aber nicht verhindern können, dass die Wäsche gerade bei diesen niedrigen Temperaturen mit Bakterien kontaminiert werden, die sich insbesondere in bestimmten Konstruktionsteilen, wie beispielsweise in der Dosiereinrichtung in Schläuchen oder in Filtern befinden und sich dort auch stark vermehren können. Diese Bakterien sind der Grund, dass Wäsche beim Trocknen "muffig" riecht, wenn diese in nicht optimal durchlüfteten Keller- oder Dachbodenräumen getrocknet wird. Beim Trocknen der Wäsche im Freien werden diese Bakterien durch die Bleichwirkung der UV-Strahlung der Sonne weitgehend abgetötet, was sich günstig für den Duft der Wäsche auswirken kann. Auch Waschmittel mit Bleichzusätzen können dies positiv beeinflussen. Allerdings setzt die Bleichwirkung erst bei Temperaturen oberhalb von typischerweise 50 bis 60°C ein, so das insbesondere bei den Niedrig-Temperatur-Waschprozessen die notwendige Desinfektionswirkung ausbleibt.

Andererseits werden zur Vermeidung einer Bakterienkontamination der Wäsche Waschmaschinen vorgeschlagen, bei denen die zirkulierende Reinigungs- und Spülflüssigkeit in einem Durchlauferhitzer einer UV-Strahlungsquelle ausgesetzt wird (DE 43 42 049). In der DE 211 00 63 wird vorgeschlagen, für eine Bleichung und Desinfektion der Wäsche in Wasch- oder Trockenmaschinen die Wäsche nach dem Wasch- bzw. Trockenvorgang einer intensiv ultravioletten Bestrahlung auszusetzen. Nachteilig ist bei derartigen Konstruktionen, dass UV-Bestrahlungen einen erhöhten Energiebedarf zur Folge haben und der Austausch der Lampen mit hohen Kosten verbunden ist. In der Regel ist der Einsatz zudem mit einer erheblichen konstruktiven Änderung des Gesamtaufbaues verbunden, was zusätzlichen Entwicklungsaufwand nach sich zieht.

Es ist Aufgabe der Erfindung, eine Waschmaschine bzw. einen Wäschetrockner der eingangs erwähnten Art zu schaffen, wobei eine Desinfektion von wasser- bzw. luftführenden Teilen mit geringem Aufwand ermöglicht ist.

Diese Aufgabe wird bei einer Waschmaschine dadurch gelösst, dass wenigstens eines der Konstruktionsteile zumindest teilweise an seiner dem luft- und/oder wasserführenden Bereich zugewandten Oberfläche antimikrobiell oder bakteriozid beschichtet ist und/oder dass diese Oberfläche antimikrobiell oder bakteriozid dotiert ist.

Die beschichteten oder dotierten Konstruktionsteile ermöglichen eine starke Eindämmung eines Bakterienwachstums oder verhindern dieses vollständig. Damit kann dann auch die Kontamination der Wäsche eingedämmt oder vollständig verhindert werden. Die biozide bzw. antimikrobielle Wirkung kann unabhängig von zusätzlichen Energiequellen (Strom, UV-Strahlung etc.) aufrechterhalten werden. Vorteilhafter Weise kann die Erfindung auch bei bestehenden Konstruktionen integriert werden. Die Konstruktionsteile müssen in ihrer geometrischen Gestalt dann nicht oder nur geringfügig abgeändert werden.

Bei einer Waschmaschine lassen sich beispielsweise eine oder mehrere der folgenden Konstruktionselemente erfindungsgemäß ausstatten:
- Waschmitteldosierbehälter;
- Zuführung zwischen dem Waschmittelbehälter und der Waschtrommel;
- Schläuche;
- Laugenpumpen;
- Laugenpumpengehäuse;
- Flusensieb;
- Waschtrommel;
- Laugenbehälter (Trommelbehälter);
- Türabdichtungen

Dies sind die Konstruktionsteile, die mit Waschwasser oder Lauge in Berührung kommen.

Bei Wäschetrocknern kann die Erfindung beispielsweise für die Gestaltung der Kunststofftrommel und/oder für die Luftführung oder ein Teil des luftführenden Systems Verwendung finden.

Gemäß einer möglichen Erfindungsvariante kann es vorgesehen sein, dass die Oberfläche wenigstens teilweise von einer Beschichtung überdeckt ist und dass die Beschichtung antimikrobielle oder bakterizide Wirkstoffe aufweist.

Die Oberflächenbeschichtung kann durch eine Pulverlackbeschichtung durch ein Siebdruck- oder ein Lackierverfahren hergestellt werden.

Eine mögliche Erfindungsausgestaltung sieht vor, dass das Konstruktionsteil zumindest teilweise von einem Kunststoffbauteil gebildet ist, und dass dem Material dieses Kunststoffbauteil eine antimikrobiell oder biozid wirkende Substanz, insbesondere ein Glaspulver zugesetzt ist.

Vorteilhaft ist, wenn das Glaspulver wenigstens teilweise von einem Ag, - Cu- und/oder Zn-dotiertem Silikat, Phosphat oder Borosilikatglas gebildet ist. Die Glaszusammensetzungen gelten als besonders antimikrobiell oder biozid.

Das Kunststoffmaterial ist bereits in seinem Rohzustand entsprechend mit der Substanz versetzt und kann zu dem gewünschten Konstruktionsteil geformt werden. Zur Formgebung sind sämtliche bekannte Verfahren anwendbar, z. B. Spritzgießen, pressen, extrudieren etc..

Es ist auch denkbar, dass das Haushaltsgerät derart gestaltet ist, dass das Konstruktionsteil wenigstens teilweise von einem Ag-, Cu- und/oder Zn-dotiertem Silikat-, Phosphat- oder Borosilikatglas gebildet ist. Das Glasmaterial bildet eine sehr glatte Oberfläche, die ein Anhaften von bakterienbildenden Substanzen erschwert. Zudem wird die Bakterienbildung aufgrund der Dotierung des Materials verhindert oder zumindest deutlich reduziert.

Eine weitere Erfindungsvariante kann dergestalt sein, dass das Konstruktionsteil einen silbergefüllten Zeolith enthält. Die silbergefüllten Zeolithe geben die wirksamen Ag⁺-lonen relativ langsam ab. Damit wird die biozide Wirkung der Konstruktionsteile lange erhalten.

Neben der Verwendung anorganischer, ist auch der Einsatz organischer biozider oder antimikrobieller Wirkstoffe denkbar. Beispielsweise kann der Wirkstoff Triclosan von Ciba Geigy als Dotierstoff für die Kunststoffteile verwendet werden.

Eine gute bakterienverhindernde Wirkung wird dann erreicht, wenn vorgesehen ist, dass die auf die Oberfläche aufgebrachte Schicht oder der dotierte Oberflächenbereich eine Konzentration von 0,5 - 10 Gew. % an antimikrobiellen oder bakterioziden Wirksubstanzen aufweist.

Da die bei den vorliegenden Anwendungen das Lebensmittelbedarfs- und Gegenständegesetz nicht beachtet werden muss, können bevorzugt wirkungsvolle Anteile größer als 3 Gew. % verwendet werden.

Die Erfindung kann auch beispielsweise bei Konstruktionsteilen in Staubsaugern, Dampfgargeräten oder dergleichen verwendet werden, wo im Gebrauch bakterielle Kontamination auftreten kann.

## Patentansprüche

1. Haushaltsgerät, insbesondere Wäschetrockner oder Waschmaschine mit einem aus einzelnen Konstruktionsteilen zusammengesetzten, luft- und/o der wasserführendem System innerhalb dem eine Waschlauge, ein Gasstrom oder dergleichen führbar ist,
**dadurch gekennzeichnet,**
**dass** wenigstens eines der Konstruktionsteile zumindest teilweise an seiner dem luft- und/oder wasserführenden Bereich zugewandten Oberfläche antimikrobiell oder bakteriozid beschichtet ist und/oder
**dass** diese Oberfläche antimikrobiell oder bakteriozid dotiert ist.

2. Haushaltsgerät nach Anspruch 1,
**dadurch gekennzeichnet,**
**dass** die Oberfläche mit organischen und/oder anorganischen bakterizid oder antimikrobiell wirkenden Substanzen beschichtet und/oder dotiert ist.

3. Haushaltsgerät nach Anspruch 1 oder 2,
**dadurch gekennzeichnet,**
**dass** die Beschichtung einen Glasanteil und/oder eine Glasfritte aufweist, die zumindest teilweise antimikrobielle und/oder biozide Wirkung aufweisen.

4. Haushaltsgeräte nach einem der Ansprüche 1 bis 3,
**dadurch gekennzeichnet,**
**dass** das Konstruktionsteil zumindest teilweise von einem Kunststoffbauteil gebildet ist, und
**dass** dem Material dieses Kunststoffbauteil eine antimikrobiell oder biozid wirkende Substanz, insbesondere ein Glaspulver zugesetzt ist.

5. Haushaltsgeräte nach einem der Ansprüche 3 oder 4,
**dadurch gekennzeichnet,**
**dass** die Glasfritte beziehungsweise das Glaspulver wenigstens teilweise von einem AG, Cu- und/oder Zn-dotierten Silikat-Phosphat- oder Borisilikatglas gebildet ist.

6. Haushaltsgerät nach einem der Ansprüche 1 bis 5,
**dadurch gekennzeichnet,**
**dass** das Konstruktionsteil wenigstens teilweise von einem Ag-, Cu- und/oder Zn-dotiertem Silikat-, Phosphat- oder Borosilikat gebildet ist.

7. Haushaltsgerät nach einem der Ansprüche 1 bis 6,
**dadurch gekennzeichnet,**
**dass** das Konstruktionsteil wenigstens teilweise einen silbergefüllten Zeolith als Beschichtungsmaterial oder als Dotierstoff aufweist.

8. Haushaltsgerät nach einem der Ansprüche 1 bis 7,
**dadurch gekennzeichnet,**
**dass** die auf die Oberfläche aufgebrachte Schicht oder der dotierte Oberflächenbereich eine Konzentration von 0,5 - 10 Gew. % an antimikrobiellen oder bakterioziden Wirksubstanzen aufweist.
